# EUROPEAN PATENT SPECIFICATION

(11) **EP 4 206 175 B1**
(45) Date of publication and mention of the grant of the patent: **29.04.2026**
(21) Application number: 21860317.3
(22) Date of filing: 23.08.2021
(51) Int. Cl.: C07C 67/31, C07C 69/736, C07C 69/618

(54) **PREPARATION OF SUBSTITUTED ACRYLATE COMPOUND**
HERSTELLUNG EINER SUBSTITUIERTEN ACRYLATVERBINDUNG
PRÉPARATION D'UN COMPOSÉ DE (MÉTH)ACRYLATE SUBSTITUÉ

(30) Priority: 24.08.2020 CN 202010856857
(43) Date of publication of application: 05.07.2023
(73) Proprietor: Hua Medicine (Shanghai) Ltd., Pudong, Shanghai 201203 (CN)
(72) Inventor: SHE, Jin, Shanghai 201203 (CN); CHEN, Li, Shanghai 201203 (CN); LV, Guanghua, Shanghai 201203 (CN); JIN, Xiangle, Shanghai 201203 (CN); XIA, Lizhen, Shanghai 201203 (CN); LI, Jun, Shanghai 201203 (CN)
(74) Representative: Cabinet Becker et Associés
(86) International application number: PCT/CN2021/114070
(87) International publication number: WO 2022/042482

(56) References cited:
- WO-A1-2019/169153
- CN-A- 109 369 471
- JP-A- H04 356 469
- JP-A- H04 356 469
- XU ZE-FENG ET AL: "Copper-Catalyzed Regio- and Stereoselective O-Arylation of Enolates", vol. 16, no. 13, 19 June 2014 (2014-06-19), US, pages 3436 - 3439, XP055904079, ISSN: 1523-7060, Retrieved from the Internet <URL:https://pubs.acs.org/doi/pdf/10.1021/ol501434d> DOI: 10.1021/ol501434d
- KAZUSHIGE TAMURA ET AL: "Synthesis of 2-polyfluoroalkylated thiochromones and chromones", JOURNAL OF FLUORINE CHEMISTRY, vol. 68, no. 1, 1 July 1994 (1994-07-01), NL, pages 25 - 31, XP055544700, ISSN: 0022-1139, DOI: 10.1016/0022-1139(93)02979-O
- MUSHTA A I ET AL: "Synthesis and some chemical properties of 1-aryloxy-2-chloro-1,2-difluoroethenes", JOURNAL OF FLUORINE CHEMISTRY, ELSEVIER, NL, vol. 126, no. 9-10, 1 October 2005 (2005-10-01), pages 1307 - 1311, XP027851805, ISSN: 0022-1139, [retrieved on 20051001]
- ROSER KURT S ET AL: "Mitochondrial biotransformation of [omega]-(phenoxy)alkanoic acids, 3-(phenoxy)acrylic acids, and [omega]-(1-methyl-1H-imidazol-2-ylthio)alkanoic acids: A prodrug strategy for targeting cytoprotective antioxidants to mito", BIOORGANIC & MEDICINAL CHEMISTRY, vol. 18, no. 4, 15 January 2010 (2010-01-15), pages 1441 - 1448, XP028649844, ISSN: 0968-0896, DOI: 10.1016/J.BMC.2010.01.019
- WAHEED MOHAMMED ET AL: "Pd/Indanone-Based Ligands: An Efficient Catalyst System for Ullmann�-Type, Suzuki-Miyaura, and Mizoroki-Heck Cross-Coupling Reactions with Aryl Tosylates and Aryl Halides", vol. 49, no. 18, 1 September 2017 (2017-09-01), STUTTGART, DE., pages 4372 - 4382, XP093199252, ISSN: 0039-7881, Retrieved from the Internet <URL:https://www.thieme-connect.de/products/ejournals/pdf/10.1055/s-0036-1589058.pdf> DOI: 10.1055/s-0036-1589058
- XU ZE-FENG, CAI CHEN-XIN, JIANG MIN, LIU JIN-TAO: "Copper-Catalyzed Regio- and Stereoselective O-Arylation of Enolates", ORGANIC LETTERS, AMERICAN CHEMICAL SOCIETY, US, vol. 16, no. 13, 3 July 2014 (2014-07-03), US , pages 3436 - 3439, XP055904079, ISSN: 1523-7060, DOI: 10.1021/ol501434d
- DATABASE REGISTRY STN; ANONYMOUS : "2-Butenoic acid, 2-methyl-3-phenoxy-, phenyl ester (CA INDEX NAME) ", XP055904082, Database accession no. RN 155530-28-2
- DATABASE REGISTRY STN; ANONYMOUS : "2-Butenoic acid, 3-(4-bromophenoxy)-2-methyl-, 4-bromophenyl ester", XP055904087, Database accession no. RN 155530-29-3
- DATABASE REGISTRY STN; ANONYMOUS : "2-Propenoic acid, 2-iodo-3-methoxy-3-(2-methylphenoxy)-, methyl ester", XP055904091, Database accession no. RN 59577-89-8

## Description

### PRIORITY CLAIM

This application claims the priority of Chinese Invention Patent Application No. 202010856857.9, filed on August 24, 2020.

### FIELD OF THE INVENTION

The present disclosure relates to a synthetic method of preparing substituted acrylate compounds of general formula (I).

### BACKGROUND OF THE INVENTION

The substituted acrylate compounds of general formula (I) are commonly used pharmaceutical intermediate compounds.

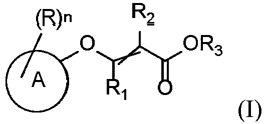

There are many methods of preparing the compound of general formula (I) in the prior art, but they generally suffer from low yields, need to use metal catalysts, poor safety, high prices of raw materials, and the like. Xu Ze-Feng et al. ("Copper-Catalyzed Regio- and Stereoselective O-Arylation of Enolates," Organic Letters, 2014, pp. 3436-3439) describes a copper-catalyzed method for the O-arylation of enolates using diaryliodonium salts as arylating agents. This synthetic approach enables the synthesis of compounds corresponding to the present formula (I).

For example, WO2013096771A1 discloses the following reaction without using a metal catalyst, which comprises reacting compound 29a with 2,5-dichlorophenol, K₂CO₃ and DMF at 120°C for 2h, with a yield of 59% (See Example 29 on pages 120-121 of the PCT publication).

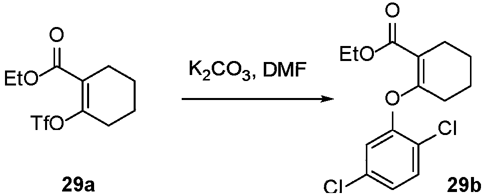

Therefore, there is still a general need for the preparation of substituted acrylate compounds of general formula (I) in an economical, safe and effective manner.

### SUMMARY OF THE INVENTION

After in-depth research, the inventors of the present invention have found a method suitable for industrial production of compounds of general formula (I), which does not use metal catalysts, can be implemented with cheap and readily available raw materials, and produces compounds of general formula (I) in high yields.

Specifically, in one aspect, the method comprises reacting a compound of general formula (II) with a compound of general formula (III), in the presence of a base: 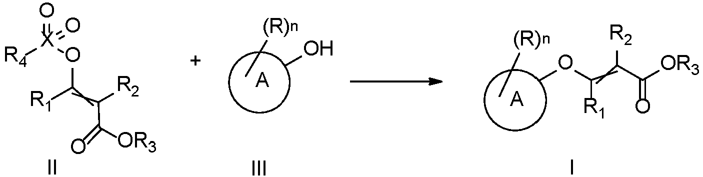 wherein
ring A is C₆₋₁₀ aryl or 5- to 10-membered heteroaryl;
X is S;
R₁ is selected from H, C₁₋₆ alkyl, C₁₋₆ haloalkyl, C₃₋₇ cycloalkyl, and 3- to 8-membered heterocyclyl;
R₂ is selected from H, C₁₋₆ alkyl, and C₁₋₆ haloalkyl;
R₃ is selected from H, C₁₋₆ alkyl, C₁₋₆ haloalkyl, C₂₋₆ alkenyl, C₂₋₆ alkynyl, -C₀₋₆ alkylene-C₃₋₇ cycloalkyl, -C₀₋₆ alkylene-3- to 8-membered heterocyclyl, -C₀₋₆ alkylene-C₆₋₁₀ aryl, and -C₀₋₆ alkylene-5- to 10-membered heteroaryl, wherein the groups are unsubstituted or independently substituted with m R' group(s);
R₄ is selected from C₁₋₂₀ alkyl, C₁₋₆ haloalkyl, -ORₐ, -NRₐR_{b}, C₃₋₇ cycloalkyl, 3- to 8-membered heterocyclyl, C₆₋₁₀ aryl, and 5- to 10-membered heteroaryl, wherein the groups are unsubstituted or independently substituted with m' R" group(s);
R is selected from H, halogen, -O-C₁₋₆ alkyl, C₁₋₆ alkyl, and C₁₋₆ haloalkyl;
n is 0, 1, 2, 3, 4, or 5;
wherein R' and R" are independently selected from halogen, -NO₂, -CN, -NRₐR_{b}, - NRₐC(O)R_{b}, C₁₋₂₀ alkyl, C₁₋₆ haloalkyl, -O-C₁₋₆ alkyl, and phenyl;
m is 1, 2, 3, 4, or 5;
m' is 1, 2, 3, 4, or 5;
Rₐ and R_{b} are independently selected from H, C₁₋₆ alkyl, C₁₋₆ haloalkyl, C₃₋₇ cycloalkyl, 3- to 8-membered heterocyclyl, C₆₋₁₀ aryl, and 5- to 10-membered heteroaryl; or Rₐ and R_{b}, together with the nitrogen atom to which they are attached, form 3- to 8-membered heterocyclyl or 5- to 10-membered heteroaryl.

In another aspect, the method does not use metal catalysts.

In another aspect, the method does not use OTf, which is the most commonly used and most reactive leaving group in the art, and can increase the reaction yield compared with using OTf.

It is further disclosed a compound of formula (I), prepared by the method described above: 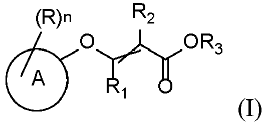 wherein
ring A is C₆₋₁₀ aryl or 5- to 10-membered heteroaryl;
R₁ is selected from H, C₁₋₆ alkyl, C₁₋₆ haloalkyl, C₃₋₇ cycloalkyl, and 3- to 8-membered heterocyclyl;
R₂ is selected from H, C₁₋₆ alkyl, and C₁₋₆ haloalkyl;
R₃ is selected from H, C₁₋₆ alkyl, C₁₋₆ haloalkyl, C₂₋₆ alkenyl, C₂₋₆ alkynyl, -C₀₋₆ alkylene-C₃₋₇ cycloalkyl, -C₀₋₆ alkylene-3- to 8-membered heterocyclyl, -C₀₋₆ alkylene-C₆₋₁₀ aryl, and -C₀₋₆ alkylene-5- to 10-membered heteroaryl, wherein the groups are unsubstituted or independently substituted with m R' group(s);
R is selected from H, halogen, -O-C₁₋₆ alkyl, C₁₋₆ alkyl, and C₁₋₆ haloalkyl;
n is 0, 1, 2, 3, 4, or 5;
wherein R' is selected from halogen, -NO₂, -CN, -NRₐR_{b}, -NRₐC(O)R_{b}, C₁₋₂₀ alkyl, C₁₋₆ haloalkyl, -O-C₁₋₆ alkyl, and phenyl;
m is 1, 2, 3, 4, or 5;
Rₐ and R_{b} are independently selected from H, C₁₋₆ alkyl, C₁₋₆ haloalkyl, C₃₋₇ cycloalkyl, 3- to 8-membered heterocyclyl, C₆₋₁₀ aryl, and 5- to 10-membered heteroaryl; or Rₐ and R_{b}, together with the nitrogen atom to which they are attached, form 3- to 8-membered heterocyclyl or 5- to 10-membered heteroaryl.

### DETAILED DESCRIPTION OF THE INVENTION

### Definition

### Chemical definitions

Definitions of specific functional groups and chemical terms are described in more detail below.

When a range of values is listed, it is intended to encompass each value and sub-range within the range. For example "C₁₋₆ alkyl" is intended to include C₁, C₂, C₃, C₄, C₅, C₆, C₁₋₆, C₁₋₅, C₁₋₄, C₁₋₃, C₁₋₂, C₂₋₆, C₂₋₅, C₂₋₄, C₂₋₃, C₃₋₆, C₃₋₅, C₃₋₄, C₄₋₆, C₄₋₅ and C₅₋₆ alkyl.

The term "about" means having a value that falls within the standard error of the accepted mean when considered by one of ordinary skill in the art. For example, "about" means ±10% of the indicated amount, or ±5% of the indicated amount.

"C₁₋₂₀ alkyl" refers to a radical of a straight or branched, saturated hydrocarbon group having 1 to 20 carbon atoms. In some embodiments, C₁₋₁₂ alkyl is alternative. In some embodiments, C₁₋₆ alkyl is alternative. In some embodiments, C₁₋₄ alkyl is alternative. Examples of C₁₋₆ alkyl include methyl (C₁), ethyl (C₂), n-propyl (C₃), iso-propyl (C₃), n-butyl (C₄), tert-butyl (C₄), sec-butyl (C₄), iso-butyl (C₄), n-pentyl (C₅), 3-pentyl (C₅), pentyl (C₅), neopentyl (C₅), 3-methyl-2-butyl (C₅), tert-pentyl (C₅) and n-hexyl (C₆). The term "C₁₋₆ alkyl" also includes heteroalkyl, wherein one or more (e.g., 1, 2, 3 or 4) carbon atoms are substituted with heteroatoms (e.g., oxygen, sulfur, nitrogen, boron, silicon, phosphorus). Alkyl groups can be optionally substituted with one or more substituents, for example, with 1 to 5 substituents, 1 to 3 substituents or 1 substituent. Conventional abbreviations of alkyl include Me (-CH₃), Et (-CH₂CH₃), iPr (-CH(CH₃)₂), nPr (-CH₂CH₂CH₃), nBu (-CH₂CH₂CH₂CH₃) or iBu (-CH_{2C}H(CH₃)₂).

"C₂₋₆ alkenyl" refers to a radical of a straight or branched hydrocarbon group having 2 to 6 carbon atoms and at least one carbon-carbon double bond. In some embodiments, C₂₋₄ alkenyl is alternative. Examples of C₂₋₆ alkenyl include vinyl (C₂), 1-propenyl (C₃), 2-propenyl (C₃), 1-butenyl (C₄), 2-butenyl (C₄), butadienyl (C₄), pentenyl (C₅), pentadienyl (C₅), hexenyl (C₆), etc. The term "C₂₋₆ alkenyl" also includes heteroalkenyl, wherein one or more (e.g., 1, 2, 3 or 4) carbon atoms are replaced by heteroatoms (e.g., oxygen, sulfur, nitrogen, boron, silicon, phosphorus). The alkenyl groups can be optionally substituted with one or more substituents, for example, with 1 to 5 substituents, 1 to 3 substituents or 1 substituent.

"C₂₋₆ alkynyl" refers to a radical of a straight or branched hydrocarbon group having 2 to 6 carbon atoms, at least one carbon-carbon triple bond and optionally one or more carbon-carbon double bonds. In some embodiments, C₂₋₄ alkynyl is alternative. Examples of C₂₋₆ alkynyl include, but are not limited to, ethynyl (C₂), 1-propynyl (C₃), 2-propynyl (C₃), 1-butynyl (C₄), 2-butynyl (C₄), pentynyl (C₅), hexynyl (C₆), etc. The term "C₂₋₆ alkynyl" also includes heteroalkynyl, wherein one or more (e.g., 1, 2, 3 or 4) carbon atoms are replaced by heteroatoms (e.g., oxygen, sulfur, nitrogen, boron, silicon, phosphorus). The alkynyl groups can be substituted with one or more substituents, for example, with 1 to 5 substituents, 1 to 3 substituents or 1 substituent.

"C₁₋₆ alkylene" refers to a divalent group of the "C₁₋₆ alkyl" as defined above, i.e., a divalent group formed by removing another hydrogen of the C₁₋₆ alkyl, and can be a substituted or unsubstituted alkylene. In some embodiments, C₁₋₄ alkylene is yet alternative. The unsubstituted alkylene groups include, but are not limited to, methylene (-CH₂-), ethylene (-CH₂CH₂-), propylene (-CH₂CH₂CH₂-), butylene (-CH₂CH₂CH₂CH₂-), pentylene (-CH₂CH₂CH₂CH₂CH₂-), hexylene (-CH₂CH₂CH₂CH₂CH₂CH₂-), etc. Examples of substituted alkylene groups, such as those substituted with one or more alkyl (methyl) groups, include, but are not limited to, substituted methylene (-CH(CH₃)-, -C(CH₃)₂-), substituted ethylene (-CH(CH₃)CH₂-, -CH₂CH(CH₃)-, -C(CH₃)₂CH₂-, -CH₂C(CH₃)₂-), substituted propylene (-CH(CH₃)CH₂CH₂-, -CH₂CH(CH₃)CH₂-, -CH₂CH₂CH(CH₃)-, -C(CH₃)₂CH₂CH₂-, - CH₂C(CH₃)₂CH₂-, -CH₂CH₂C(CH₃)₂-), etc.

"C₀₋₆ alkylene" refers to a chemical bond and "C₁₋₆ alkylene".

"Halo" or "halogen" refers to fluorine (F), chlorine (Cl), bromine (Br) and iodine (I).

Thus, "C₁₋₆ haloalkyl" refers to the above "C₁₋₆ alkyl", which is substituted by one or more halogens. In some embodiments, C₁₋₄ haloalkyl is yet alternative, and still alternatively C₁₋₂ haloalkyl. Exemplary haloalkyl groups include, but are not limited to, -CF₃, -CH₂F, -CHF₂, -CHFCH₂F, -CH₂CHF₂, -CF₂CF₃, -CCl₃, -CH₂Cl, -CHCl₂, 2,2,2-trifluoro-1,1-dimethyl-ethyl, and the like. The haloalkyl can be substituted at any available point of attachment, for example, with 1 to 5 substituents, 1 to 3 substituents or 1 substituent.

"C₃₋₇ cycloalkyl" refers to a radical of a non-aromatic cyclic hydrocarbon group having from 3 to 7 ring carbon atoms and zero heteroatom. In some embodiments, C₃₋₅ cycloalkyl is alternative. In other embodiments, C₃₋₆ cycloalkyl is alternative. In other embodiments, C₅₋₆ cycloalkyl is alternative. The cycloalkyl also includes a ring system in which the cycloalkyl described herein is fused with one or more aryl or heteroaryl groups, wherein the point of attachment is on the cycloalkyl ring, and in such case, the number of carbon atoms continues to represent the number of carbon atoms in the cycloalkyl system. Exemplary cycloalkyl groups include, but are not limited to, cyclopropyl (C₃), cyclopropenyl (C₃), cyclobutyl (C₄), cyclobutenyl (C₄), cyclopentyl (C₅), cyclopentenyl (C₅), cyclohexyl (C₆), cyclohexenyl (C₆), cyclohexadienyl (C₆), cycloheptyl (C₇), cycloheptenyl (C₇), cycloheptadienyl (C₇), cycloheptatrienyl (C₇), etc. The cycloalkyl can be substituted with one or more substituents, for example, with 1 to 5 substituents, 1 to 3 substituents or 1 substituent.

"3- to 8-membered heterocyclyl" refers to a radical of 3- to 8-membered non-aromatic ring system having ring carbon atoms and 1 to 4 ring heteroatoms. 3- to 6-membered heterocyclyl is alternative, which is a radical of 3- to 6-membered non-aromatic ring system having ring carbon atoms and 1 to 3 ring heteroatoms. 3- to 5-membered heterocyclyl is alternative, which is a radical of 3- to 5-membered non-aromatic ring system having ring carbon atoms and 1 to 2 ring heteroatoms. 4- to 8-membered heterocyclyl is alternative, which is a radical of 4- to 8-membered non-aromatic ring system having ring carbon atoms and 1 to 3 ring heteroatoms. 5- to 6-membered heterocyclyl is yet alternative, which is a radical of 5- to 6-membered non-aromatic ring system having ring carbon atoms and 1 to 3 ring heteroatoms. The heterocyclyl also includes a ring system wherein the heterocyclyl described above is fused with one or more cycloalkyl groups, wherein the point of attachment is on the cycloalkyl ring, or the heterocyclyl described above is fused with one or more aryl or heteroaryl groups, wherein the point of attachment is on the heterocyclyl ring; and in such cases, the number of ring members continues to represent the number of ring members in the heterocyclyl ring system. Exemplary 3-membered heterocyclyl groups containing one heteroatom include, but are not limited to, aziridinyl, oxiranyl and thiiranyl (thiorenyl). Exemplary 4-membered heterocyclyl groups containing one heteroatom include, but are not limited to, azetidinyl, oxetanyl and thietanyl. Exemplary 5-membered heterocyclyl groups containing one heteroatom include, but are not limited to, tetrahydrofuranyl, dihydrofuranyl, tetrahydrothiophenyl, dihydrothienyl, pyrrolidinyl, dihydropyrrolyl and pyrrolyl-2,5-dione. Exemplary 5-membered heterocyclyl groups containing two heteroatoms include, but are not limited to, dioxolanyl, oxasulfuranyl, disulfuranyl, and oxazolidin-2-one. Exemplary 5-membered heterocyclyl groups containing three heteroatoms include, but are not limited to, triazolinyl, oxadiazolinyl, and thiadiazolinyl. Exemplary 6-membered heterocyclyl groups containing one heteroatom include, but are not limited to, piperidyl, tetrahydropyranyl, dihydropyridyl and thianyl. Exemplary 6-membered heterocyclyl groups containing two heteroatoms include, but are not limited to, piperazinyl, morpholinyl, dithianyl and dioxanyl. Exemplary 6-membered heterocyclyl groups containing three heteroatoms include, but are not limited to, triazinanyl. Exemplary 7-membered heterocycly groups containing one heteroatom include, but are not limited to, azepanyl, oxepanyl and thiepanyl. Exemplary 5-membered heterocyclyl groups fused with a C₆ aryl (also referred as 5,6-bicyclic heterocyclyl herein) include, but are not limited to, indolinyl, isoindolinyl, dihydrobenzofuranyl, dihydrobenzothiophenyl, benzoxazolinonyl, etc. Exemplary 6-membered heterocyclyl groups fused with a C₆ aryl (also referred as 6,6-bicyclic heterocyclyl herein) include, but are not limited to, tetrahydroquinolinyl, tetrahydroisoquinolinyl, etc. The heterocyclyl can be substituted with one or more substituents, for example, with 1 to 5 substituents, 1 to 3 substituents or 1 substituent.

"C₆₋₁₀ aryl" refers to a radical of monocyclic or polycyclic (e.g., bicyclic) 4n+2 aromatic ring system having 6-10 ring carbon atoms and zero heteroatom (e.g., having 6 or 10 shared π electrons in a cyclic array). In some embodiments, the aryl group has six ring carbon atoms ("C₆ aryl"; for example, phenyl). In some embodiments, the aryl group has ten ring carbon atoms ("C₁₀ aryl"; for example, naphthyl, e.g., 1-naphthyl and 2-naphthyl). The aryl group also includes a ring system in which the aryl ring described above is fused with one or more cycloalkyl or heterocyclyl groups, and the point of attachment is on the aryl ring, in which case the number of carbon atoms continues to represent the number of carbon atoms in the aryl ring system. The aryl can be substituted with one or more substituents, for example, with 1 to 5 substituents, 1 to 3 substituents or 1 substituent.

"5- to 10-membered heteroaryl" refers to a radical of 5- to 10-membered monocyclic or bicyclic 4n+2 aromatic ring system (e.g., having 6 or 10 shared π electrons in a cyclic array) having ring carbon atoms and 1-4 ring heteroatoms, wherein each heteroatom is independently selected from nitrogen, oxygen and sulfur. In the heteroaryl group containing one or more nitrogen atoms, the point of attachment can be a carbon or nitrogen atom as long as the valence permits. Heteroaryl bicyclic systems may include one or more heteroatoms in one or two rings. Heteroaryl also includes ring systems wherein the heteroaryl ring described above is fused with one or more cycloalkyl or heterocyclyl groups, and the point of attachment is on the heteroaryl ring. In such case, the number the carbon atoms continues to represent the number of carbon atoms in the heteroaryl ring system. In some embodiments, 5- to 6-membered heteroaryl groups are yet alternative, which are radicals of 5- to 6-membered monocyclic or bicyclic 4n+2 aromatic ring systems having ring carbon atoms and 1-4 ring heteroatoms. Exemplary 5-membered heteroaryl groups containing one heteroatom include, but are not limited to, pyrrolyl, furyl and thienyl. Exemplary 5-membered heteroaryl groups containing two heteroatoms include, but are not limited to, imidazolyl, pyrazolyl, oxazolyl, isoxazolyl, thiazolyl, and isothiazolyl. Exemplary 5-membered heteroaryl groups containing three heteroatoms include, but are not limited to, triazolyl, oxadiazolyl (such as, 1,2,4- oxadiazoly), and thiadiazolyl. Exemplary 5-membered heteroaryl groups containing four heteroatoms include, but are not limited to, tetrazolyl. Exemplary 6-membered heteroaryl groups containing one heteroatom include, but are not limited to, pyridyl. Exemplary 6-membered heteroaryl groups containing two heteroatoms include, but are not limited to, pyridazinyl, pyrimidinyl, and pyrazinyl. Exemplary 6-membered heteroaryl groups containing three or four heteroatoms include, but are not limited to, triazinyl and tetrazinyl, respectively. Exemplary 7-membered heteroaryl groups containing one heteroatom include, but are not limited to, azepinyl, oxepinyl, and thiepinyl. Exemplary 5,6-bicyclic heteroaryl groups include, but are not limited to, indolyl, isoindolyl, indazolyl, benzotriazolyl, benzothiophenyl, isobenzothiophenyl, benzofuranyl, benzoisofuranyl, benzimidazolyl, benzoxazolyl, benzoisoxazolyl, benzoxadiazolyl, benzothiazolyl, benzoisothiazolyl, benzothiadiazolyl, indolizinyl and purinyl. Exemplary 6,6-bicyclic heteroaryl groups include, but are not limited to, naphthyridinyl, pteridinyl, quinolyl, isoquinolyl, cinnolinyl, quinoxalinyl, phthalazinyl and quinazolinyl. The heteroaryl can be substituted with one or more substituents, for example, with 1 to 5 substituents, 1 to 3 substituents or 1 substituent.

Specific examples of alternative heteroaryl groups include: pyrrolyl, imidazolyl, pyrazolyl, 2-pyridyl, 3-pyridyl, 4-pyridyl, pyrimidinyl, pyrazinyl, pyridazinyl, triazolyl (4H-1,2,4-triazolyl, 1H-1,2,3-triazolyl, 2H-1,2,3-triazolyl, pyranyl, 2-furyl, 3-furyl, 2-thienyl, 3-thienyl, oxazolyl, isoxazolyl, oxazolyl (1,2,4-oxazolyl, 1,3,4-oxazolyl, 1,2,5-oxazolyl, thiazolyl, thiadiazolyl (1,2,4-thiadiazolyl, 1,3,4-thiadiazolyl, 1,2,5-thiadiazolyl).

Alkyl, alkenyl, alkynyl, carbocyclyl, heterocyclyl, aryl and heteroaryl as defined herein are optionally substituted groups.

Exemplary substituents on carbon atoms include, but are not limited to, halogen, -CN, -NO₂, -N₃, -SO₂H, -SO₃H, -OH, -OR^{aa}, -ON(R^{bb})₂, -N(R^{bb})₂, -N(R^{bb})₃⁺X⁻, -N(OR^{cc})R^{bb}, -SH, - SR^{aa}, -SSR^{cc}, -C( = O)R^{aa}, -CO₂H, -CHO, -C(OR^{cc})₂, -CO₂R^{aa}, -OC( = O)R^{aa}, -OCO₂R^{aa}, -C( = O)N(R^{bb})₂, -OC( = O)N(R^{bb})₂, -NR^{bb}C( = O)R^{aa}, -NR^{bb}CO₂R^{aa}, -NR^{bb}C( = O)N(R^{bb})₂, -C( = NR^{bb})R^{aa}, -C( = NR^{bb})OR^{aa}, -OC( = NR^{bb})R^{aa}, -OC( = NR^{bb})OR^{aa}, -C( = NR^{bb})N(R^{bb})₂, -OC( = NR^{bb})N(R^{bb})₂, -NR^{bb}C( = NR^{bb})N(R^{bb})₂, -C( = O)NR^{bb}SO₂R^{aa}, -NR^{bb}SO₂R^{aa}, -SO₂N(R^{bb})₂,-SO₂R^{aa}, -SO₂OR^{aa}, -OSO₂R^{aa}, -S( = O)R^{aa}, -OS( = O)R^{aa}, -Si(R^{aa})₃, -OSi(R^{aa})₃, -C( = S)N(R^{bb})₂, -C( = O)SR^{aa}, -C( = S)SR^{aa}, -SC( = S)SR^{aa}, -SC( = O)SR^{aa}, -OC( = O)SR^{aa}, -SC( = O)OR^{aa}, - SC( = O)R^{aa}, -P( = O)₂R^{aa}, -OP( = O)₂R^{aa}, -P( = O)(R^{aa})₂, -OP( = O)(R^{aa})₂, -OP( = O)(OR^{cc})₂, -P( = O)₂N(R^{bb})₂, -OP( = O)₂N(R^{bb})₂, -P( = O)(NRb^{bb})₂, -OP( = O)(NR^{bb})₂, -NR^{bb}P( = O)(OR^{cc})₂, -NR^{bb}P( = O)(NR^{bb})₂, -P(R^{cc})₂, -P(R^{cc})₃, -OP(R^{cc})₂, -OP(R^{cc})₃, -B(R^{aa})₂, -B(OR^{cc})₂, -BR^{aa}(OR^{cc}), alkyl, haloalkyl, alkenyl, alkynyl, carbocyclyl, heterocyclyl, aryl and heteroaryl, wherein each of the alkyl, alkenyl, alkynyl, carbocyclyl, heterocyclyl, aryl and heteroaryl is independently substituted with 0, 1, 2, 3, 4 or 5 R^{dd} groups;
or two geminal hydrogens on a carbon atom are replaced with = O, = S, = NN(R^{bb})₂, = NNR^{bb}C( = O)R^{aa}, = NNR^{bb}C( = O)OR^{aa}, = NNR^{bb}S( = O)₂R^{aa}, = NR^{bb} or = NOR^{cc} groups;
each of the R^{aa} is independently selected from alkyl, haloalkyl, alkenyl, alkynyl, carbocyclyl, heterocyclyl, aryl and heteroaryl, or two of the R^{aa} groups are combined to form a heterocyclyl or heteroaryl ring, wherein each of the alkyl, alkenyl, alkynyl, carbocyclyl, heterocyclyl, aryl and heteroaryl is independently substituted with 0, 1, 2, 3, 4 or 5 R^{dd} groups;
each of the R^{bb} is independently selected from hydrogen, -OH, -OR^{aa}, -N(R^{cc})₂, -CN, -C( = O)R^{aa}, -C( = O)N(R^{cc})₂, -CO₂R^{aa}, -SO₂R^{aa}, -C( = NR^{cc})OR^{aa}, -C( = NR^{cc})N(R^{cc})₂, - SO₂N(R^{cc})₂, -SO₂R^{cc}, -SO₂OR^{cc}, -SOR^{aa}, -C( = S)N(R^{cc})₂, -C( = O)SR^{cc}, -C( = S)SR^{cc}, -P( = O)₂R^{aa}, -P( = O)(R^{aa})₂, -P( = O)₂N(R^{cc})₂, -P( = O)(NR^{cc})₂, alkyl, haloalkyl, alkenyl, alkynyl, carbocyclyl, heterocyclyl, aryl and heteroaryl, or two R^{bb} groups are combined to form a heterocyclyl or a heteroaryl ring, wherein each of the alkyl, alkenyl, alkynyl, carbocyclyl, heterocyclyl, aryl and heteroaryl is independently substituted with 0, 1, 2, 3, 4 or 5 R^{dd} groups;
each of the R^{cc} is independently selected from hydrogen, alkyl, haloalkyl, alkenyl, alkynyl, carbocyclyl, heterocyclyl, aryl and heteroaryl, or two R^{cc} groups are combined to form a heterocyclyl or a heteroaryl ring, wherein each of the alkyl, alkenyl, alkynyl, carbocyclyl, heterocyclyl, aryl and heteroaryl is independently substituted with 0, 1, 2, 3, 4 or 5 R^{dd} groups;
each of the R^{dd} is independently selected from halogen, -CN, -NO₂, -N₃, -SO₂H, - SO₃H, -OH, -OR^{ee}, -ON(R^{ff})₂, -N(R^{ff})₂, -N(R^{ff})₃⁺X⁻, -N(OR^{ee})R^{ff}, -SH, -SR^{ee}, -SSR^{ee}, -C( = O)R^{ee}, -CO₂H, -CO₂R^{ee}, -OC( = O)R^{ee}, -OCO₂R^{ee}, -C( = O)N(R^{ff})₂, -OC( = O)N(R^{ff})₂, - NR^{ff}C( = O)R^{ee}, -NR^{ff}CO₂R^{ee}, -NR^{ff}C( = O)N(R^{ff})₂, -C( = NR^{ff})OR^{ee}, -OC( = NR^{ff})R^{ee}, -OC( = NR^{ff})OR^{ee}, -C( = NR^{ff})N(R^{ff})₂, -OC( = NR^{ff})N(R^{ff})₂, -NR^{ff}C( = NR^{ff})N(R^{ff})₂, -NR^{ff}SO₂R^{ee}, - SO₂N(R^{ff})₂, -SO₂R^{ee}, -SO₂OR^{ee}, -OSO₂R^{ee}, -S( = O)R^{ee}, -Si(R^{ee})₃, -OSi(R^{ee})₃, -C( = S)N(R^{ff})₂, -C( = O)SR^{ee}, -C( = S)SR^{ee}, -SC( = S)SR^{ee}, -P( = O)₂R^{ee}, -P( = O)(R^{ee})₂, -OP( = O)(R^{ee})₂, - OP( = O)(OR^{ee})₂, alkyl, haloalkyl, alkenyl, alkynyl, carbocyclyl, heterocyclyl, aryl, heteroaryl, wherein each of the alkyl, alkenyl, alkynyl, carbocyclyl, heterocyclyl, aryl and heteroaryl is independently substituted with 0, 1, 2, 3, 4 or 5 R²⁸ groups, or two geminal R^{dd} substituents can be combined to form = O or = S;
each of the R^{ee} is independently selected from alkyl, haloalkyl, alkenyl, alkynyl, carbocyclyl, aryl, heterocyclyl, and heteroaryl, wherein each of the alkyl, alkenyl, alkynyl, carbocyclyl, heterocyclyl, aryl and heteroaryl is independently substituted with 0, 1, 2, 3, 4 or 5 R^{gg} groups;
each of the R^{ff} is independently selected from hydrogen, alkyl, haloalkyl, alkenyl, alkynyl, carbocyclyl, heterocyclyl, aryl and heteroaryl, or two R^{ff} groups are combined to form a heterocyclyl or a heteroaryl ring, wherein each of the alkyl, alkenyl, alkynyl, carbocyclyl, heterocyclyl, aryl and heteroaryl is independently substituted with 0, 1, 2, 3, 4 or 5 R^{gg} groups;
each of the R^{gg} is independently selected from halogen, -CN, -NO₂, -N₃, -SO₂H, - SO₃H, -OH, -OC₁₋₆ alkyl, -ON(C₁₋₆ alkyl)₂, -N(C₁₋₆ alkyl)₂, -N(C₁₋₆ alkyl)₃⁺X⁻, -NH(C₁₋₆ alkyl)₂⁺X⁻, -NH₂(C₁₋₆ alkyl)⁺X⁻, -NH₃⁺X⁻, -N(OC₁₋₆ alkyl)(C₁₋₆ alkyl), -N(OH)(C₁₋₆ alkyl), - NH(OH), -SH, -SC₁₋₆ alkyl, -SS(C₁₋₆ alkyl), -C( = O)(C₁₋₆ alkyl), -CO₂H, -CO₂(C₁₋₆ alkyl), - OC( = O)(C₁₋₆ alkyl), -OCO₂(C₁₋₆ alkyl), -C( = O)NH₂, -C( = O)N(C₁₋₆ alkyl)₂, -OC( = O)NH(C₁₋₆ alkyl), -NHC( = O)(C₁₋₆ alkyl), -N(C₁₋₆ alkyl)C( = O)(C₁₋₆ alkyl), -NHCO₂(C₁₋₆ alkyl), -NHC( = O)N(C₁₋₆ alkyl)₂, -NHC( = O)NH(C₁₋₆ alkyl), -NHC( = O)NH₂, -C( = NH)O(C₁₋₆ alkyl), -OC( = NH)(C₁₋₆ alkyl), -OC( = NH)OC₁₋₆ alkyl, -C( = NH)N(C₁₋₆ alkyl)₂, - C(= NH)NH(C₁₋₆ alkyl), -C( = NH)NH₂, -OC( = NH)N(C₁₋₆ alkyl)₂, -OC(NH)NH(C₁₋₆ alkyl), -OC(NH)NH₂, -NHC(NH)N(C₁₋₆ alkyl)₂, -NHC( = NH)NH₂, -NHSO₂(C₁₋₆ alkyl), -SO₂N(C₁₋₆ alkyl)₂, -SO₂NH(C₁₋₆ alkyl), -SO₂NH₂, -SO₂C₁₋₆ alkyl, -SO₂OC₁₋₆ alkyl, -OSO₂C₁₋₆ alkyl, - SOC₁₋₆ alkyl, -Si(C₁₋₆ alkyl)₃, -OSi(C₁₋₆ alkyl)₃, -C( = S)N(C₁₋₆ alkyl)₂, C( = S)NH(C₁₋₆ alkyl), C( = S)NH₂, -C( = O)S(C₁₋₆ alkyl), -C( = S)SC₁₋₆ alkyl, -SC( = S)SC₁₋₆ alkyl, -P( = O)₂(C₁₋₆ alkyl), -P( = O)(C₁₋₆ alkyl)₂, -OP( = O)(C₁₋₆ alkyl)₂, -OP( = O)(OC₁₋₆ alkyl)₂, C₁₋₆ alkyl, C₁₋₆ haloalkyl, C₂-C₆ alkenyl, C₂-C₆ alkynyl, C₃-C₇ carbocyclyl, C₆-C₁₀ aryl, C₃-C₇ heterocyclyl, C₅-C₁₀ heteroaryl; or two geminal R^{gg} substituents may combine to form = O or = S; wherein X⁻ is a counter-ion.

Exemplary substituents on nitrogen atoms include, but are not limited to, hydrogen, - OH, -OR^{aa}, -N(R^{cc})₂, -CN, -C( = O)R^{aa}, -C( = O)N(R^{cc})2, -CO₂R^{aa}, -SO₂R^{aa}, -C( = NR^{bb})R^{aa}, - C( = NR^{cc})OR^{aa}, -C(=NR^{cc})N(R^{cc})₂, -SO₂N(R^{cc})₂, -SO₂R^{cc}, -SO₂OR^{cc}, -SOR^{aa}, -C(= S)N(R^{cc})₂, -C( = O)SR^{cc}, -C( = S)SR^{cc}, -P( = O)₂R^{aa}, -P( = O)(R^{aa})₂, -P( = O)₂N(R^{cc})₂, -P( = O)(NR^{cc})₂, alkyl, haloalkyl, alkenyl, alkynyl, carbocyclyl, heterocyclyl, aryl and heteroaryl, or two R^{cc} groups attached to a nitrogen atom combine to form a heterocyclyl or a heteroaryl ring, wherein each of the alkyl, alkenyl, alkynyl, carbocyclyl, heterocyclyl, aryl and heteroaryl is independently substituted with 0, 1, 2, 3, 4 or 5 R^{dd} groups, and wherein R^{aa}, R^{bb}, R^{cc} and R^{dd} are as described herein.

### SPECIFIC EMBODIMENTS

In one embodiment, the present disclosure relates to a method of preparing a compound of general formula (I), comprising reacting a compound of general formula (II) with a compound of general formula (III), in the presence of a base: 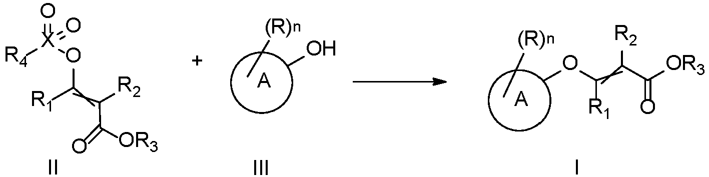 wherein
ring A is C₆₋₁₀ aryl or 5- to 10-membered heteroaryl;
X is S;
R₁ is selected from H, C₁₋₆ alkyl, C₁₋₆ haloalkyl, C₃₋₇ cycloalkyl, and 3- to 8-membered heterocyclyl;
R₂ is selected from H, C₁₋₆ alkyl, and C₁₋₆ haloalkyl;
R₃ is selected from H, C₁₋₆ alkyl, C₁₋₆ haloalkyl, C₂₋₆ alkenyl, C₂₋₆ alkynyl, -C₀₋₆ alkylene-C₃₋₇ cycloalkyl, -C₀₋₆ alkylene-3- to 8-membered heterocyclyl, -C₀₋₆ alkylene-C₆₋₁₀ aryl, and -C₀₋₆ alkylene-5- to 10-membered heteroaryl, wherein the groups are unsubstituted or independently substituted with m R' group(s);
R₄ is selected from C₁₋₂₀ alkyl, C₁₋₆ haloalkyl, -ORₐ, -NRₐR_{b}, C₃₋₇ cycloalkyl, 3- to 8-membered heterocyclyl, C₆₋₁₀ aryl, and 5- to 10-membered heteroaryl, wherein the groups are unsubstituted or independently substituted with m' R" group(s);
R is selected from H, halogen, -O-C₁₋₆ alkyl, C₁₋₆ alkyl, and C₁₋₆ haloalkyl;
n is 0, 1, 2, 3, 4, or 5;
wherein R' and R" are independently selected from halogen, -NO₂, -CN, -NRₐR_{b}, - NRₐC(O)R_{b}, C₁₋₂₀ alkyl, C₁₋₆ haloalkyl, -O-C₁₋₆ alkyl, and phenyl;
m is 1, 2, 3, 4, or 5;
m' is 1, 2, 3, 4, or 5;
Rₐ and R_{b} are independently selected from H, C₁₋₆ alkyl, C₁₋₆ haloalkyl, C₃₋₇ cycloalkyl, 3- to 8-membered heterocyclyl, C₆₋₁₀ aryl, and 5- to 10-membered heteroaryl; or Rₐ and R_{b}, together with the nitrogen atom to which they are attached, form 3- to 8-membered heterocyclyl or 5- to 10-membered heteroaryl.

### Ring A

In a specific embodiment, ring A is C₆₋₁₀ aryl; in another specific embodiment, ring A is 5- to 10-membered heteroaryl; in another specific embodiment, ring A is phenyl; in another specific embodiment, ring A is naphthyl; in another specific embodiment, ring A is 5- to 6-membered heteroaryl; in another specific embodiment, ring A is thienyl.

### R₁

In a specific embodiment, R₁ is H; in another specific embodiment, R₁ is C₁₋₆ alkyl; in another specific embodiment, R₁ is methyl; in another specific embodiment, R₁ is C₁₋₆ haloalkyl; in another specific embodiment, R₁ is C₃₋₇ cycloalkyl; in another specific embodiment, R₁ is 3-to 8-membered heterocyclyl.

### R₂

In a specific embodiment, R₂ is H; in another specific embodiment, R₂ is C₁₋₆ alkyl; in another specific embodiment, R₂ is C₁₋₆ haloalkyl.

### R₃

In a specific embodiment, R₃ is H; in another specific embodiment, R₃ is C₁₋₆ alkyl; in another specific embodiment, R₃ is ethyl; in another specific embodiment, R₃ is t-butyl; in another specific embodiment, R₃ is C₁₋₆ haloalkyl; in another specific embodiment, R₃ is C₂₋₆ alkenyl; in another specific embodiment, R₃ is C₂₋₆ alkynyl; in another specific embodiment, R₃ is -C₀₋₆ alkylene-C₃₋₇ cycloalkyl; in another specific embodiment, R₃ is -C₀₋₆ alkylene-3- to 8-membered heterocyclyl; in another specific embodiment, R₃ is -C₀₋₆ alkylene-C₆₋₁₀ aryl; in another specific embodiment, R₃ is -C₀₋₆ alkylene-5- to 10-membered heteroaryl; in another specific embodiment, R₃ is benzyl. In the above specific embodiments, the groups are unsubstituted or independently substituted with m R' group(s).

### R₄

In a specific embodiment, R₄ is C₁₋₂₀ alkyl; in another specific embodiment, R₄ is C₁₋₁₂ alkyl; in another specific embodiment, R₄ is C₁₋₆ alkyl; in another specific embodiment, R₄ is C₁₋₆ haloalkyl; in another specific embodiment, R₄ is -ORₐ; in another specific embodiment, R₄ is methyl; in another specific embodiment, R₄ is -NRₐR_{b}; in another specific embodiment, R₄ is C₃₋₇ cycloalkyl; in another specific embodiment, R₄ is 3- to 8-membered heterocyclyl; in another specific embodiment, R₄ is C₆₋₁₀ aryl; in another specific embodiment, R₄ is phenyl; in another specific embodiment, R₄ is 5- to 10-membered heteroaryl. In the above specific embodiments, the groups are unsubstituted or independently substituted with m' R" group(s).

### R' and R"

In a specific embodiment, R' is halogen; in another specific embodiment, R' is chloro; in another specific embodiment, R' is -NO₂; in another specific embodiment, R' is -CN; in another specific embodiment, R' is -NRₐR_{b}; in another specific embodiment, R' is -NRₐC(O)R_{b}; in another specific embodiment, R' is C₁₋₂₀ alkyl; in another specific embodiment, R' is C₁₋₁₂ alkyl; in another specific embodiment, R' is C₁₋₆ alkyl; in another specific embodiment, R' is methyl; in another specific embodiment, R' is C₁₋₆ haloalkyl; in another specific embodiment, R' is -O-C₁₋₆ alkyl; in another specific embodiment, R' is phenyl.

In a specific embodiment, R" is halogen; in another specific embodiment, R" is chloro; in another specific embodiment, R" is -NO₂; in another specific embodiment, R" is -CN; in another specific embodiment, R" is -NRₐR_{b}; in another specific embodiment, R" is - NRₐC(O)R_{b}; in another specific embodiment, R" is C₁₋₂₀ alkyl; in another specific embodiment, R" is C₁₋₁₂ alkyl; in another specific embodiment, R" is C₁₋₆ alkyl; in another specific embodiment, R" is methyl; in another specific embodiment, R" is C₁₋₆ haloalkyl; in another specific embodiment, R" is -O-C₁₋₆ alkyl; in another specific embodiment, R" is phenyl.

In the above specific embodiments, Rₐ and R_{b} are independently selected from H, C₁₋₆ alkyl and C₁₋₆ haloalkyl.

### m

In a specific embodiment, m is 0; in another specific embodiment, m is 1; in another specific embodiment, m is 2; in another specific embodiment, m is 3; in another specific embodiment, m is 4; in another specific embodiment, m is 5.

### m'

In a specific embodiment, m' is 0; in another specific embodiment, m' is 1; in another specific embodiment, m' is 2; in another specific embodiment, m' is 3; in another specific embodiment, m' is 4; in another specific embodiment, m' is 5.

### R

In a specific embodiment, R is H; in another specific embodiment, R is halogen; in another specific embodiment, R is chloro; in another specific embodiment, R is -O-C₁₋₆ alkyl; in another specific embodiment, R is C₁₋₆ alkyl; in another specific embodiment, R is C₁₋₆ haloalkyl.

### n

In a specific embodiment, n is 0; in another specific embodiment, n is 1; in another specific embodiment, n is 2; in another specific embodiment, n is 3; in another specific embodiment, n is 4; in another specific embodiment, n is 5.

Any technical solution or any combination thereof in any one of the above specific embodiments may be combined with any technical solution or any combination thereof in other specific embodiments. For example, any technical solution or any combination thereof of X may be combined with any technical solution or any combination thereof of ring A, R₁-R₄, R, R', R", m, m', n, and the like. The present disclosure is intended to include all the combinations of such technical solutions, which are not exhaustively listed here to save space.

In a more specific embodiment, the present disclosure provides the above method, wherein R₄ is C₆₋₁₀ aryl or 5- to 10-membered heteroaryl, which is unsubstituted or independently substituted with m' R" group(s).

In a more specific embodiment, the present disclosure provides the above method, wherein R₄ is selected from phenyl, 1-naphthyl, 2-naphthyl, 2-chlorophenyl, 3-chlorophenyl, 4-chlorophenyl, 2-bromophenyl, 3-bromophenyl, 4-bromophenyl, 2-fluorophenyl, 3-fluorophenyl, 4-fluorophenyl, 2,6-dichlorophenyl, 2,4-difluorophenyl, 3-cyanophenyl, 4-cyanophenyl, 2-nitrophenyl, 4-nitrophenyl, 2-methylphenyl, 4-methylphenyl, 4-propylphenyl, 4-t-butylphenyl, 2,5-dimethylphenyl, mesityl, 2,4,6-triisopropylphenyl, 2-dodecylphenyl, 3-dodecylphenyl, 4-dodecylphenyl, 2-(trifluoromethyl)phenyl, 3-(trifluoromethyl)phenyl, 3,5-bis(trifluoromethyl)phenyl, 4-methoxyphenyl, N,N-dimethylaminophenyl, 4-acetylaminophenyl, 4-biphenyl, thienyl and 5-bromothienyl; alternatively, R₄ is selected from phenyl, 1-naphthyl, 2-naphthyl, 4-chlorophenyl, 3-fluorophenyl, 4-fluorophenyl, 4-nitrophenyl, 2-methylphenyl, 4-propylphenyl, 4-t-butylphenyl, 2,5-dimethylphenyl, 2,4,6-triisopropylphenyl, 2-dodecylphenyl, 3-dodecylphenyl, 4-dodecylphenyl, 4-methoxyphenyl, 4-acetylaminophenyl, 4-methylphenyl and mesityl; yet alternatively, R₄ is selected from phenyl, 4-chlorophenyl, 4-methylphenyl and mesityl.

In a more specific embodiment, the present disclosure provides the above method, wherein R₄ is C₁₋₁₂ alkyl, -ORₐ, -NRₐR_{b}, C₃₋₇ cycloalkyl, or 3- to 8-membered heterocyclyl, which is unsubstituted or independently substituted with m' R" group(s).

In a more specific embodiment, the present disclosure provides the above method, wherein R₄ is selected from N,N-dimethylamino, methyl, ethyl, butyl, dodecyl, methoxy, ethoxy, and cyclopropyl.

In a more specific embodiment, the present disclosure provides the above method, wherein ring A is substituted or unsubstituted phenyl, naphthyl or thienyl; alternatively substituted or unsubstituted phenyl.

In a more specific embodiment, the present disclosure provides the above method, wherein R is H, halogen, C₁₋₆ alkyl, or C₁₋₆ haloalkyl; alternatively, R is H or halogen.

In a more specific embodiment, the present disclosure provides the above method, wherein 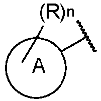 is selected from phenyl, naphthalene-1-yl, naphthalene-2-yl, 2-chlorophenyl, 3-chlorophenyl, 4-chlorophenyl, 2-bromophenyl, 3-bromophenyl, 4-bromophenyl, 2,3-dichlorophenyl, 2,4-dichlorophenyl, 2,5-dichlorophenyl, 3,4-dichlorophenyl, 2,3,5-trichlorophenyl, 4-isopropylphenyl and 4-t-butylphenyl; alternatively, 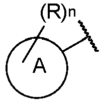 is 2-chlorophenyl.

In a more specific embodiment, the present disclosure provides the above method, wherein R₁ is selected from C₁₋₆ alkyl, such as methyl.

In a more specific embodiment, the present disclosure provides the above method, wherein R₂ is H.

In a more specific embodiment, the present disclosure provides the above method, wherein R₃ is H, -CH₃, -CH₂CH₃, -CH(CH₃)₂, -C(CH₃)₃, -CH₂CH₂CH₂CH₃, phenyl or -CH₂-phenyl; alternatively, R₃ is -CH₃, -CH₂CH₃, -C(CH₃)₃ or -CH₂-phenyl.

In a more specific embodiment, the present disclosure provides the above method, wherein the base is selected from inorganic bases and organic bases; alternatively, the inorganic base is selected from carbonates, bicarbonates, phosphates, hydrogenphosphates, dihydrogenphosphates, hydroxides and hydrides of alkali metals and alkaline earth metals, for example, LiOH, NaOH, KOH, Li₂CO₃, Na₂CO₃, K₂CO₃, Cs₂CO₃, Na₃PO₄, K₃PO₄, K₂HPO₄, KH₂PO₄ and NaH; alternatively, the organic base is selected from alkoxides of alkali metals and alkaline earth metals, and organic amines, such as NaOMe, KOMe, NaOEt, KOEt, NaOtBu, KOtBu, triethylamine, DBU (1,8-diazabicyclo[5.4.0]undec-7-ene), DIPEA (N,N-diisopropylethylamine), DABCO (1,4-diazabicyclo[2.2.2]octane) and DMAP (4-dimethylaminopyridine).

In a more specific embodiment, the present disclosure provides the above method, wherein the reaction is performed in the presence of a solvent selected from water, alkanes, ethers, esters, alcohols, halogenated hydrocarbons, ketones, amides, sulfones, sulfoxides, nitriles, and mixtures thereof, such as water, n-heptane, toluene, THF (tetrahydrofuran), MTBE (methyl tert-butyl ether), methyltetrahydrofuran, ethyl acetate, isopropyl acetate, butyl acetate, methanol, ethanol, isopropanol, tert-butanol, tert-amyl alcohol, dichloromethane, 1,2-dichloroethane, chlorobenzene, acetone, 2-butanone, DMF (N,N-dimethylformamide), DMA (N,N-dimethylacetamide), NMP (N-methylpyrrolidone), DMSO (dimethyl sulfoxide) and acetonitrile.

In a more specific embodiment, the present disclosure provides the above method, wherein the reaction temperature is from room temperature to the reflux temperature of the solvent, alternatively 40-90°C; yet alternatively 50-80°C.

In a more specific embodiment, the present disclosure provides the above method, wherein the molar ratio of the compound of the general formula (II) to the compound of the general formula (III) is 1:(0.7-3), alternatively 1:(0.7-1.5), yet alternatively 1:(1-1.2), still alternatively 1:1, 1:1.05, 1:1.1, 1:1.15, or 1:1.2.

In a more specific embodiment, the present disclosure provides the above method, wherein the molar ratio of the compound of the general formula (II) to the base is 1:(1-5), alternatively 1:(2-4), yet alternatively 1:1.5, 1:1.6, 1:1.7, 1:1.8, 1:1.9, 1:2, 1:2.1, 1:2.2, 1:2.3, 1:2.4, 1:2.5, 1:2.6, 1:2.7, 1:2.8, 1:2.9, or 1:3.

In a more specific embodiment, the method of the present disclosure is implemented on an industrial scale.

It is further disclosed a compound of formula (I), prepared by the method described above: 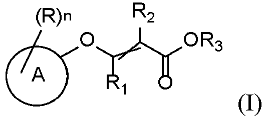 wherein
ring A is C₆₋₁₀ aryl or 5- to 10-membered heteroaryl;
R₁ is selected from H, C₁₋₆ alkyl, C₁₋₆ haloalkyl, C₃₋₇ cycloalkyl, and 3- to 8-membered heterocyclyl;
R₂ is selected from H, C₁₋₆ alkyl, and C₁₋₆ haloalkyl;
R₃ is selected from H, C₁₋₆ alkyl, C₁₋₆ haloalkyl, C₂₋₆ alkenyl, C₂₋₆ alkynyl, -C₀₋₆ alkylene-C₃₋₇ cycloalkyl, -C₀₋₆ alkylene-3- to 8-membered heterocyclyl, -C₀₋₆ alkylene-C₆₋₁₀ aryl, and -C₀₋₆ alkylene-5- to 10-membered heteroaryl, wherein the groups are unsubstituted or independently substituted with m R' group(s);
R is selected from H, halogen, -O-C₁₋₆ alkyl, C₁₋₆ alkyl, and C₁₋₆ haloalkyl;
n is 0, 1, 2, 3, 4, or 5;
wherein R' is selected from halogen, -NO₂, -CN, -NRₐR_{b}, -NRₐC(O)R_{b}, C₁₋₂₀ alkyl, C₁₋₆ haloalkyl, -O-C₁₋₆ alkyl, and phenyl;
m is 1, 2, 3, 4, or 5;
Rₐ and R_{b} are independently selected from H, C₁₋₆ alkyl, C₁₋₆ haloalkyl, C₃₋₇ cycloalkyl, 3- to 8-membered heterocyclyl, C₆₋₁₀ aryl, and 5- to 10-membered heteroaryl; or Rₐ and R_{b}, together with the nitrogen atom to which they are attached, form 3- to 8-membered heterocyclyl or 5- to 10-membered heteroaryl.

### Examples

Materials or reagents used herein are either commercially available or prepared by synthetic methods generally known in the art. The following reaction scheme exemplarily illustrates the practice of the method for the compound of the present disclosure.

### General operation

The reaction scheme and typical operation of preparing the compound of general formula (I) are as follows:

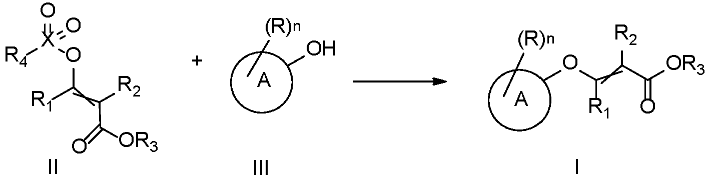

To a reactor are added the compound of general formula (III), a base, the compound of general formula (II), and an optional solvent, and the temperature is maintained at room temperature to reflux temperature of the solvent. The mixture is stirred until the reaction is completed, during which the progress of the reaction is monitored. After completion of the reaction, the reaction solution is cooled down to room temperature. The organic phase is separated and purified to give the compound of general formula (I).

The compound of general formula (II) is commercially available, or is prepared by a method known in the art. For example, the following route can be used:

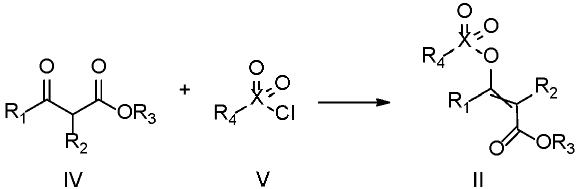

To a reactor are added the compound of general formula (IV), the compound of general formula (V), a base and a solvent. The mixture is stirred at a certain temperature until the reaction is completed, during which the progress of the reaction is monitored. After completion of the reaction, the organic phase is separated. The organic phase can be directly used for the next reaction step, or evaporated to dryness for later use, or purified by rectification or column chromatography.

Alternatively, the compound of general formula (V) can be converted into an acid anhydride before reacted with the compound of general formula (IV) as described above.

### Example 1

### Preparation of ethyl 3-(2-chlorophenoxy)-2-butenoate

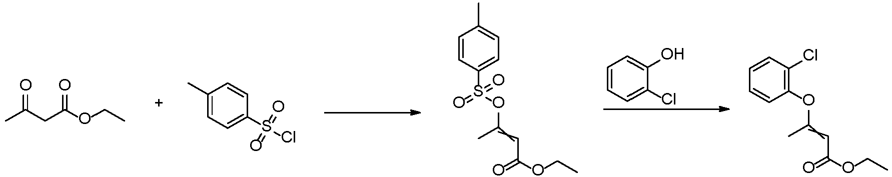

(1) Ethyl acetoacetate (50.0 g, 384 mmol, 1.0 eq), triethylamine (38.8 g, 384 mmol, 1 eq), DABCO (17.2 g, 154 mmol, 0.4 eq), and 250 mL of isopropyl acetate were added to a reactor, and then a solution of p-toluenesulfonyl chloride (87.8 g, 460.8 mmol, 1.2 eq) in isopropyl acetate (250 mL) was added dropwise at 0-10 °C over 1-2 h. After the addition was completed, the mixture was reacted with stirring at 10-20 °C for 3 h. After the reaction was completed, 250 mL of water was added to quench the reaction. The organic phase was separated and washed respectively with 250 mL of 5% p-toluenesulfonic acid solution and 250 mL of 8% sodium bicarbonate solution to give a solution of ethyl 3-(p-toluenesulfonyloxy)-2-butenoate in isopropyl acetate (98.7% yield (external standard method)), which was used after evaporation to dryness (99.2% purity) or directly for the next reaction.
(2) Potassium carbonate (150 g, 1.09 mol, 2.8 eq), 350 mL of isopropyl acetate, and the solution of ethyl 3-(p-toluenesulfonyloxy)-2-butenoate in isopropyl acetate from step (1) were added to a reactor. The mixture was heated to 70-80 °C, and then a solution of 2-chlorophenol (50 g, 0.389 mol, 1.0 eq) in isopropyl acetate (400 mL) was added dropwise at 70-80 °C over 2-3 h. After the addition was completed, the mixture was reacted with stirring at 70-80 °C for another 19 h. The reaction mixture was cooled down to room temperature and quenched by adding water. The organic phase was separated as a solution of ethyl 3-(2-chlorophenoxy)-2-butenoate in isopropyl acetate with a purity of 94.9%. The above solution of ethyl 3-(2-chlorophenoxy)-2-butenoate in isopropyl acetate was concentrated to give 99.7 g of crude product (containing 85.4% product), with a total yield of 92.1% over two steps.

¹H-NMR (CDCl₃, 400 MHz): δ 7.46-7.48(m, 1H), 7.29-7.33(m, 1H), 7.21-7.23(m, 1H), 7.10-7.11(m, 1H), 4.78(s, 1H), 4.11(q, 2H, J = 4.2), 2.54(s, 3H), 1.23(t, 3H, J = 4.2).

### Examples 2-15

The corresponding compounds were prepared according to the method of Example 1, using the reagents or groups in the table below.

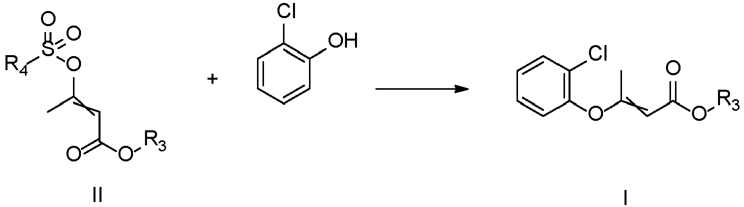

| **Example** | **Structure of** | **R₃** | **Base** | **Solvent** | **Temperature and time** | **Yield** |
|---|---|---|---|---|---|---|
| | 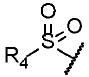 | | | | | |
| 2 | 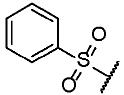 | Et | K₂CO₃, 1.9 eq | IPrOAc + DMF = 2:1 | 70-80 °C, 16 h | 94.3% |
| 3 | 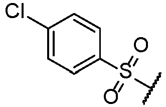 | Et | K₂CO₃, 1.9 eq | IPrOAc + DMF = 2:1 | 70-80 °C, 16 h | 73.7% over two steps |
| 4 | 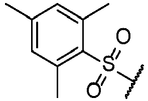 | Et | K₂CO₃, 1.9 eq | IPrOAc + DMF = 2:1 | 70-80 °C, 16 h | 96.3% |
| 5 | 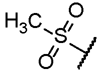 | Et | K₂CO₃, 2.0 eq | IPrOAc + DMF = 2:1 | 60-70 °C, 16 h | 73.6% |
| 6 | 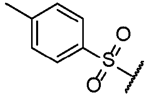 | Bn | K₂CO₃, 2.0 eq | IPrOAc | 70-80 °C, 16 h | 89.3% |
| 7 | 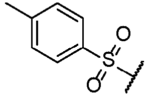 | Et | K₃PO₄·3H₂O, 2.0 eq | IPrOAc | 50-60 °C, 20 h | 82.3% |
| 8 | 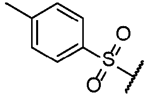 | Et | NaOH, 2.0 eq | IPrOAc | 50-60 °C, 16 h | 88.9% |
| 9 | 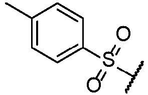 | Et | K₃PO₄, 2.0 eq | IPrOAc | 50-60 °C, 16 h | 91.6% |
| 10 | 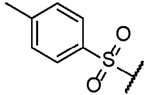 | Et | DABCO, 2.0 eq | IPrOAc | 50-60 °C, 42 h | 90.2% |
| 11 | 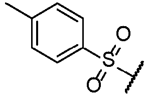 | Et | K₂CO₃, 2.0 eq | n-Hep | 60-70 °C, 16 h | 70.7% |
| 12 | 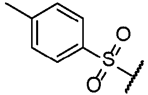 | Et | K₂CO₃, 2.0 eq | Me-THF | 60-70 °C, 16 h | 97.3% |
| 13 | 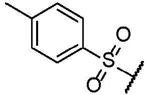 | Et | K₂CO₃, 1.9 eq | DMF | 70-80 °C, 15 h | 90.5% |
| 14 | 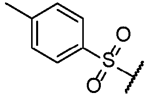 | Et | K₂CO₃, 3.0 eq | tert-Amyl alcohol | 80 °C, 16 h | 85.5% |
| 15 | 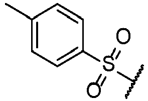 | Et | K₂CO₃, 1.9 eq | 2-Butanone | 60-70 °C, 16 h | 97.6% |

| | | | | | | |
|---|---|---|---|---|---|---|
| Notes: IPrOAc: isopropyl acetate; DMF: N,N-dimethylformamide; Bn: benzyl; DABCO: 1,4-diazabicyclo[2.2.2]octane; n-Hep: n-heptane; Me-THF: 2-methyltetrahydrofuran. | | | | | | |

Example 6: tert-Butyl 3-(2-chlorophenoxy)-2-butenoate, ¹H-NMR (CDCl₃, 400 MHz): δ7.36-7.38(m, 1H), 7.19-7.21(m, 1H), 7.10-7.11(m, 1H), 7.01-7.03(m, 1H), 4.61(s, 1H), 2.46(s, 3H), 1.35(s, 9H).

Example 7: Benzyl 3-(2-chlorophenoxy)-2-butenoate, ¹H-NMR (CDCl₃, 400 MHz): δ7.33-7.35(m, 1H), 7.20-7.32(m, 6H), 7.17-7.21(m, 1H), 7.00-7.10(m, 1H), 4.99(s, 2H), 4.72(s, 1H), 2.46(s, 3H).

## Claims

1. A method of preparing a compound of general formula (I), comprising reacting a compound of general formula (II) with a compound of general formula (III), in the presence of a base: 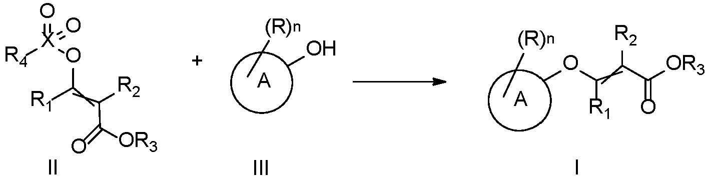 wherein
ring A is C₆₋₁₀ aryl or 5- to 10-membered heteroaryl;
X is S;
R₁ is selected from H, C₁₋₆ alkyl, C₁₋₆ haloalkyl, C₃₋₇ cycloalkyl, and 3- to 8-membered heterocyclyl;
R₂ is selected from H, C₁₋₆ alkyl, and C₁₋₆ haloalkyl;
R₃ is selected from H, C₁₋₆ alkyl, C₁₋₆ haloalkyl, C₂₋₆ alkenyl, C₂₋₆ alkynyl, -C₀₋₆ alkylene-C₃₋₇ cycloalkyl, -C₀₋₆ alkylene-3- to 8-membered heterocyclyl, -C₀₋₆ alkylene-C₆₋₁₀ aryl, and -C₀₋₆ alkylene-5- to 10-membered heteroaryl, wherein the groups are unsubstituted or independently substituted with m R' group(s);
R₄ is selected from C₁₋₂₀ alkyl, C₁₋₆ haloalkyl, -ORₐ, -NRₐR_{b}, C₃₋₇ cycloalkyl, 3- to 8-membered heterocyclyl, C₆₋₁₀ aryl, and 5- to 10-membered heteroaryl, wherein the groups are unsubstituted or independently substituted with m' R" group(s);
R is selected from H, halogen, -O-C₁₋₆ alkyl, C₁₋₆ alkyl, and C₁₋₆ haloalkyl;
n is 0, 1, 2, 3, 4, or 5;
wherein R' and R'' are independently selected from halogen, -NO₂, -CN, -NRₐR_{b}, -NRₐC(O)R_{b}, C₁₋₂₀ alkyl, C₁₋₆ haloalkyl, -O-C₁₋₆ alkyl, and phenyl;
m is 1, 2, 3, 4, or 5;
m' is 1, 2, 3, 4, or 5;
Rₐ and R_{b} are independently selected from H, C₁₋₆ alkyl, C₁₋₆ haloalkyl, C₃₋₇ cycloalkyl, 3- to 8-membered heterocyclyl, C₆₋₁₀ aryl, and 5- to 10-membered heteroaryl; or Rₐ and R_{b}, together with the nitrogen atom to which they are attached, form 3- to 8-membered heterocyclyl or 5-to 10-membered heteroaryl.

2. The method of claim 1, wherein R₄ is C₆₋₁₀ aryl or 5- to 10-membered heteroaryl, which is unsubstituted or independently substituted with m' R" group(s); alternatively, wherein R₄ is selected from phenyl, 1-naphthyl, 2-naphthyl, 2-chlorophenyl, 3-chlorophenyl, 4-chlorophenyl, 2-bromophenyl, 3-bromophenyl, 4-bromophenyl, 2-fluorophenyl, 3-fluorophenyl, 4-fluorophenyl, 2,6-dichlorophenyl, 2,4-difluorophenyl, 3-cyanophenyl, 4-cyanophenyl, 2-nitrophenyl, 4-nitrophenyl, 2-methylphenyl, 4-methylphenyl, 4-propylphenyl, 4-t-butylphenyl, 2,5-dimethylphenyl, mesityl, 2,4,6-triisopropylphenyl, 2-dodecylphenyl, 3-dodecylphenyl, 4-dodecylphenyl, 2-(trifluoromethyl)phenyl, 3-(trifluoromethyl)phenyl, 3,5-bis(trifluoromethyl)phenyl, 4-methoxyphenyl, N,N-dimethylaminophenyl, 4-acetylaminophenyl, 4-biphenyl, thienyl and 5-bromothienyl; alternatively, R₄ is selected from phenyl, 1-naphthyl, 2-naphthyl, 4-chlorophenyl, 3-fluorophenyl, 4-fluorophenyl, 4-nitrophenyl, 2-methylphenyl, 4-propylphenyl, 4-t-butylphenyl, 2,5-dimethylphenyl, 2,4,6-triisopropylphenyl, 2-dodecylphenyl, 3-dodecylphenyl, 4-dodecylphenyl, 4-methoxyphenyl, 4-acetylaminophenyl, 4-methylphenyl and mesityl; yet alternatively, R₄ is selected from phenyl, 4-chlorophenyl, 4-methylphenyl and mesityl;
alternatively, wherein R₄ is C₁₋₁₂ alkyl, -ORₐ, -NRₐR_{b}, C₃₋₇ cycloalkyl, or 3- to 8-membered heterocyclyl, which is unsubstituted or independently substituted with m' R" group(s); alternatively, wherein R₄ is selected from N,N-dimethylamino, methyl, ethyl, butyl, dodecyl, methoxy, ethoxy, and cyclopropyl.

3. The method of claim 1 or 2, wherein ring A is substituted or unsubstituted phenyl, naphthyl or thienyl; alternatively substituted or unsubstituted phenyl.

4. The method of any one of claims 1-3, wherein R is H, halogen, C₁₋₆ alkyl, or C₁₋₆ haloalkyl; alternatively, R is H or halogen.

5. The method of any one of claims 1-4, wherein 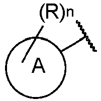 is selected from phenyl, naphthalene-1-yl, naphthalene-2-yl, 2-chlorophenyl, 3-chlorophenyl, 4-chlorophenyl, 2-bromophenyl, 3-bromophenyl, 4-bromophenyl, 2,3-dichlorophenyl, 2,4-dichlorophenyl, 2,5-dichlorophenyl, 3,4-dichlorophenyl, 2,3,5-trichlorophenyl, 4-isopropylphenyl and 4-t-butylphenyl; alternatively, 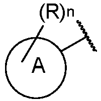 is 2-chlorophenyl.

6. The method of any one of claims 1-5, wherein R₁ is selected from C₁₋₆ alkyl, such as methyl.

7. The method of any one of claims 1-6, wherein R₂ is H.

8. The method of any one of claims 1-7, wherein R₃ is H, -CH₃, -CH₂CH₃, -CH(CH₃)₂, - C(CH₃)₃, -CH₂CH₂CH₂CH₃, phenyl or -CH₂-phenyl; alternatively, R₃ is -CH₃, -CH₂CH₃, - C(CH₃)₃ or -CH₂-phenyl.

9. The method of any one of claims 1-8, wherein the base is selected from inorganic bases and organic bases; alternatively, the inorganic base is selected from carbonates, bicarbonates, phosphates, hydrogenphosphates, dihydrogenphosphates, hydroxides and hydrides of alkali metals and alkaline earth metals, for example, LiOH, NaOH, KOH, Li₂CO₃, Na₂CO₃, K₂CO₃, Cs₂CO₃, Na₃PO₄, K₃PO₄, K₂HPO₄, KH₂PO₄ and NaH; alternatively, the organic base is selected from alkoxides of alkali metals and alkaline earth metals and organic amines, such as NaOMe, KOMe, NaOEt, KOEt, NaOtBu, KOtBu, triethylamine, DBU, DIPEA, DABCO and DMAP.

10. The method of any one of claims 1-9, wherein the reaction is performed in the presence of a solvent selected from water, alkanes, ethers, esters, alcohols, halogenated hydrocarbons, ketones, amides, sulfones, sulfoxides, nitriles, and mixtures thereof, such as water, n-heptane, toluene, THF, MTBE, methyltetrahydrofuran, ethyl acetate, isopropyl acetate, butyl acetate, methanol, ethanol, isopropanol, tert-butanol, tert-amyl alcohol, dichloromethane, 1,2-dichloroethane, chlorobenzene, acetone, 2-butanone, DMF, DMA, NMP, DMSO and acetonitrile.

11. The method of any one of claims 1-10, wherein the reaction temperature is from room temperature to the reflux temperature of the solvent, alternatively 40-90°C; yet alternatively 50-80°C.

12. The method of any one of claims 1-11, wherein the molar ratio of the compound of the general formula (II) to the compound of the general formula (III) is 1:(0.7-3), alternatively 1:(0.7-1.5), yet alternatively 1:(1-1.2), still alternatively 1:1, 1:1.05, 1:1.1, 1:1.15 or 1:1.2.

13. The method of any one of claims 1-12, wherein the molar ratio of the compound of the general formula (II) to the base is 1:(1-5), alternatively 1:(2-4), yet alternatively 1:1.5, 1:1.6, 1:1.7, 1:1.8, 1:1.9, 1:2, 1:2.1, 1:2.2, 1:2.3, 1:2.4, 1:2.5, 1:2.6, 1:2.7, 1:2.8, 1:2.9 or 1:3.

## Patentansprüche

1. Ein Verfahren zur Herstellung einer Verbindung der allgemeinen Formel (I) umfassend Reagieren eine Verbindung der allgemeinen Formel (II) mit einer Verbindung der allgemeinen Formel (III), in der Anwesenheit einer Base: 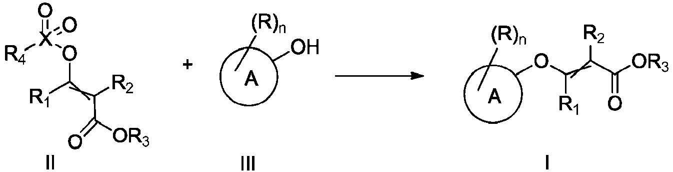 wobei
Ring A C₆₋₁₀ Aryl oder 5- bis 10-gliedriges Heteroaryl ist;
X S ist;
R₁ ausgewählt ist aus H, C₁₋₆ Alkyl, C₁₋₆ Haloalkyl, C₃₋₇ Cycloalkyl und 3- bis 8-gliedriges Heterocyclyl;
R₂ ausgewählt ist aus H, C₁₋₆ Alkyl, und C₁₋₆ Haloalkyl;
R₃ ausgewählt ist aus H, C₁₋₆ Alkyl, C₁₋₆ Haloalkyl, C₂₋₆ Alkenyl, C₂₋₆ Alkynyl, -C₀₋₆ Alkylen-C₃₋₇-Cycloalkyl, -C₀₋₆ Aklylen- 3- bis 8- gliedriges Heterocyclyl, -C₀₋₆ Alkylen-C₆₋₁₀ Aryl, und -C₀₋₆ Alkylen-5- bis 10- gliedriges Heteroaryl, wobei die Gruppen unsubstituiert oder unabhängig voneineander substituiert mit m R' Gruppe(n) sind;
R₄ ausgewählt ist aus C₁₋₂₀ Alkyl, C₁₋₆ Haloalkyl, -ORₐ, -NRₐR_{b}, C₃₋₇ Cycloalkyl, 3- bis 8-gliedriges Heterocyclyl, C₆₋₁₀ Aryl und 5- bis 10- gliedriges Heteroaryl, wobei die Gruppen unsubstituiert oder unabhängig voneineander substituiert mit m' R''Gruppe(n) sind;
R ausgewählt ist aus H, Halogen, -O-C₁₋₆ Alkyl, C₁₋₆ Alkyl und C₁₋₆ Haloalkyl;
n 0, 1, 2, 3, 4 oder 5 ist;
wobei R' und R'' unabhängig ausgewählt sind aus Halogen, -NO₂, -CN, -NRₐR_{b}, - NRₐC(O)R_{b}, C₁₋₂₀ Alkyl, C₁₋₆ Haloalkyl, -O-C₁₋₆ Alkyl, und Phenyl;
m 1, 2, 3, 4, oder 5 ist,
m' 1, 2, 3, 4, oder 5 ist;
Rₐ und R_{b} unabgängig voneinander ausgewählt sind aus H, C₁₋₆ Alkyl, C₁₋₆ Haloalkyl, C₃₋₇ Cycloalkyl, 3- bis 8-gliedriges Heterocyclyl, C₆₋₁₀ Aryl und 5- bis 10-gliedriges Heteroaryl; oder Rₐ und R_{b} bilden zusammen mit dem Stickstoffatom, an das sie gebunden sind, 3- bis 8-gliedriges Heterocyclyl oder 5- bis 10 gliedriges Heteroaryl.

2. Das Verfahren gemäß Anspruch 1, wobei R₄ C₆₋₁₀ Aryl oder 5- bis 10- gliedriges Heteroaryl ist, das unsubstituiert oder unabhängig voneinander mit m' R'' Gruppe(n) substituiert sind; alternativ, wobei R₄ ausgewählt ist aus Phenyl, 1-Naphthyl, 2-Naphthyl, 2-Chlorphenyl, 3-Chlorphenyl, 4-Chlorphenyl, 2-Bromphenyl, 3-Bromphenyl, 4-Bromphenyl, 2-Fluorphenyl, 3-Fluorphenyl, 4-Fluorphenyl, 2,6-Dichlorphenyl, 2,4-Difluorphenyl, 3-Cyanophenyl, 4-Cyanophenyl, 2-Nitrophenyl, 4-Nitrophenyl, 2-Methylphenyl, 4-Methylphenyl, 4-Propylphenyl, 4-t-Butylphenyl, 2,5-Dimethylphenyl, Mesityl, 2,4,6-Triisoprpylphenyl, 2-Dodecyphenyl, 3-Dodecylphenyl, 4-Dodecylphenyl, 2-(Trifluormethyl)phenyl, 3-(Trifluormethyl)phenyl, 3,5-Bis(trisfluormethyl)phenyl, 4-Methoxyphenyl, N,N-Dimethylaminophenyl, 4-Acetylaminophenyl, 4-Biphenyl, Thienyl und 5-Bromthienyl, alternativ ist R₄ ausgewählt aus Phenyl, 1-Naphthyl, 2-Naphthyl, 4-Chlorphenyl, 3-Fluorphenyl, 4-Fluorphenyl, 4-Nitrophenyl, 2-Methylphenyl, 4-Propylphenyl, 4-t-Butylphenyl, 2,5-Dimethylphenyl, 2,4,6-Trisisopropylphenyl, 2-Dodecylphenyl, 3-Dodecylphenyl, 4-Dodecylphenyl, 4-Methoxyphenyl, 4-Acetylaminophenyl, 4-Methylphenyl und Mesityl, oder alternativ ist R₄ ausgewählt aus Phenyl, 4-Chlorphenyl, 4-Methylphenyl und Mesityl;
alternativ, wobei R₄ C₁₋₁₂ Alkyl, -ORa, -NRₐR_{b}, C₃₋₇ Cycloalkyl oder 3- bis 8-gliedriges Heterocyclyl ist, das unsubstituiert oder unabhängig voneinander mit m' R'' Gruppe(n) substituiert ist;
alternativ, wobei R₄ ausgewählt ist aus N,N-Dimethylamino, Methyl, Ethyl, Butyl, Dodecyl, Methoxy, Ethoxy und Cyclopropyl.

3. Das Verfahren gemäß Anspruch 1 oder 2, wobei Rind A substituiertes oder unsubstituiertes Phenyl, Naphthyl oder Thienyl ist, alternativ substituiertes oder unsubstituiertes Phenyl.

4. Das Verfahren gemäß einem der Ansprüche 1 bis 3, wobei R H, Halogen, C₁₋₆ Alkyl oder C₁₋₆ Haloalkyl ist; alternativ R ist H oder Halogen.

5. Das Verfahren gemäß einem der Ansprüche 1 bis 4, wobei 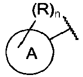 ausgewählt ist aus Phenyl, Naphthalen-1-yl, Naphthalen-2-yl, 2-Chlorphenyl, 3-Chlorphenyl, 4-Chlorphenyl, 2-Bromphenyl, 3-Bromphenyl, 4-Bromphenyl, 2,3-Dichlorphenyl, 2,4-Dichlorphenyl, 2,5-Dichlorphenyl, 3,4-Dichlorphenyl, 2,3,5-Trichlorphenyl, 4-Isopropylphenyl und 4-t-Butylphenyl, alternativ, 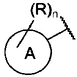 ist 2-Chlorphenyl.

6. Das Verfahren gemäß einem der Ansprüche 1 bis 5, wobei R₁ ausgewählt ist aus C₁₋₆ Alkyl, wie z.B. Methyl.

7. Das Verfahren gemäß einem der Ansprüche 1 bis 6, wobei R₂ H ist.

8. Das Verfahren gemäß einem der Ansprüche 1 bis 7, wobei R₃ H, -CH₃, -CH₂CH₃, - CH(CH₃)₂, -C(CH₃)₃, -CH₂CH₂CH₂CH₃, Phenyl oder -CH₂-Phenyl ist, alternativ ist R₃ - CH₃, -CH₂CH₃, C(CH₃)₃ oder -CH₂-Phenyl.

9. Das Verfahren gemäß einem der Ansprüche 1 bis 8, wobei die Base ausgewählt ist aus anorganischen Basen und organischen Basen; alternativ ist die anorganische Base ausgewählt aus Karbonate, Bikarbonate, Phosphate, Hydrogenphosphate, Dihydrogenphosphate, Hydroxide und Hydride von Alkalimetallen und Erdalkalimetallen, zum Beispiel, LiOH, NaOH, KOH, Li₂CO₃, Na₂CO₃, K₂CO₃, Cs₂CO₃, Na₃PO₄, K₃PO₄, K₂HPO₄, KH₂PO₄ und NaH; alternativ ist die organische Base ausgewählt aus Alkoxide von Alkalimetallen und Erdalkalimetallen und organischem Aminen, wie z.B: NaOMe, KOMe, NaOEt, KOEt, NaOtBu, KOtBu, Triethylamin, DBU, DIPEA, DABCO und DMAP.

10. Das Verfahren gemäß einem der Ansprüche 1 bis 9, wobei die Reaktion durchgeführt wird in Anwesenheit eines Lösungsmittels ausgewählt aus Wasser, Alkane, Ethern, Estern, Alkohole, halogenierte Kohlenwasserstoffe, Ketone, Amide, Sulfone, Sulfoxide, Nitrile und Gemische davon, wie zum Beispiel Wasser, n-Heptan, Toluen, THF, MTBE, Methyltetrahydrofuran, Ethylacetat, Isopropylacetat, Butylacetat, Methanol, Ethanol, Isopropanol, tert-Butanol, tert-Amyl Alkohol, Dichlormethan, 1,2-Dichlorethan, Chlorbenzen, Aceton, 2-Butanon, DMF, DMA, NMP, DMSO und Acetonitril.

11. Das Verfahren gemäß einem der Ansprüche 1 bis 10, wobei die Reaktionstemperatur von Raumtemperatur bis zur Rückflusstemperatur des Lösungsmittels ist, alternativ 40-90°C, oder alternativ 50-80°C.

12. Das Verfahren gemäß einem der Ansprüche 1 bis 11, wobei das Molverhältnis der Verbindung der allgemeinen Formel (II) zu der Verbindung der allgemeinen Formel (III) 1:(0,7-3) ist, alternativ 1:(0,7-1,5), oder alternativ 1:(1-1,2), oder noch alternativ 1:1, 1:1,05, 1:1,1, 1:1,15 oder 1:1,2.

13. Das Verfahren gemäß einem der Ansprüche 1 bis 12, wobei das Molverhältnis der Verbindung der allgemeinen Formel (II) zur Base 1:(1-5) ist, alternativ 1:(2-4), oder alternativ 1:1,5, 1:1,6, 1:1,7, 1:1,8, 1:1,9, 1:2, 1:2,1, 1:2,2, 1:2,3, 1:2,4, 1:2,5, 1:2,6, 1:2,7, 1:2,8, 1:2,9 oder 1:3.

## Revendications

1. Méthode de préparation d'un composé de formule générale (I), comprenant la réaction d'un composé de formule générale (II) avec un composé de formule générale (III), en la présence d'une base : 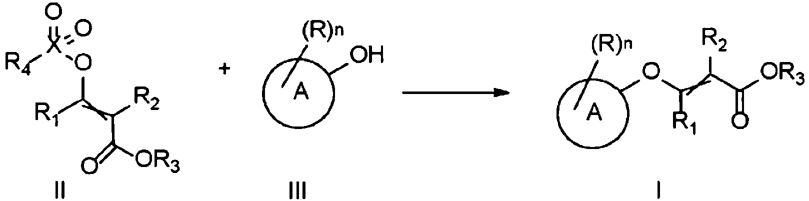 dans lesquelles :
le cycle A est un aryle en C₆₋₁₀ ou un hétéroaryle de 5 à 10 chaînons ;
X est S ;
R₁ est choisi parmi H, alkyle en C₁₋₆, halogénoalkyle en C₁₋₆, cycloalkyle en C₃₋₇, et hétérocyclyle de 3 à 8 chaînons ;
R₂ est choisi parmi H, alkyle en C₁₋₆, et halogénoalkyle en C₁₋₆ ;
R₃ est choisi parmi H, alkyle en C₁₋₆, halogénoalkyle en C₁₋₆, alcényle en C₂₋₆, alcynyle en C₂₋₆, -alkylène en C₀₋₆-cycloalkyle en C₃₋₇, -alkylène en C₀₋₆-hétérocyclyle de 3 à 8 chaînons, -alkylène en C₀₋₆-aryle en C₆₋₁₀, et -alkylène en C₀₋₆-hétéroaryle de 5 à 10 chaînons, où les groupes sont non substitués ou substitués indépendamment par m groupe(s) R' ;
R₄ est choisi parmi alkyle en C₁₋₂₀, halogénoalkyle en C₁₋₆, -ORₐ, -NRₐR_{b}, cycloalkyle en C₃₋₇, hétérocyclyle de 3 à 8 chaînons, aryle en C₆₋₁₀, et hétéroaryle de 5 à 10 chaînons, où les groupes sont non substitués ou substitués indépendamment par m' groupe(s) R" ;
R est choisi parmi H, halogène, -O-alkyle en C₁₋₆, alkyle en C₁₋₆, et halogénoalkyle en C₁₋₆ ;
n est égal à 0, 1, 2, 3, 4, ou 5 ;
où R' et R" sont choisis indépendamment parmi halogène, -NO₂, -CN, -NRₐR_{b}, -NRₐC(O)R_{b}, alkyle en C₁₋₂₀, halogénoalkyle en C₁₋₆, -O-alkyle en C₁₋₆, et phényle ;
m est égal à 1, 2, 3, 4 ou 5 ;
m' est égal à 1, 2, 3, 4 ou 5 ;
Rₐ et R_{b} sont choisis indépendamment parmi H, alkyle en C₁₋₆, halogénoalkyle en C₁₋₆, cycloalkyle en C₃₋₇, hétérocyclyle de 3 à 8 chaînons, aryle en C₆₋₁₀, et hétéroaryle de 5 à 10 chaînons ; ou Rₐ et R_{b}, conjointement avec l'atome d'azote auquel ils sont reliés, forment un hétérocyclyle de 3 à 8 chaînons ou un hétéroaryle de 5 à 10 chaînons.

2. Méthode selon la revendication 1, dans laquelle R₄ est un aryle en C₆₋₁₀ ou un hétéroaryle de 5 à 10 chaînons, qui est non substitué ou substitué indépendamment par m' groupe(s) R" ; en variante, dans laquelle R₄ est choisi parmi le phényle, le 1-naphtyle, le 2-naphtyle, le 2-chlorophényle, le 3-chlorophényle, le 4-chlorophényle, le 2-bromophényle, le 3-bromophényle, le 4-bromophényle, le 2-fluorophényle, le 3-fluorophényle, le 4-fluorophényle, le 2,6-dichlorophényle, le 2,4-difluorophényle, le 3-cyanophényle, le 4-cyanophényle, le 2-nitrophényle, le 4-nitrophényle, le 2-méthylphényle, le 4-méthylphényle, le 4-propylphényle, le 4-tert-butylphényle, le 2,5-diméthylphényle, le mésityle, le 2,4,6-triisopropylphényle, le 2-dodécylphényle, le 3-dodécylphényle, le 4-dodécylphényle, le 2-(trifluorométhyl)phényle, le 3-(trifluorométhyl)phényle, le 3,5-bis(trifluorométhyl)phényle, le 4-méthoxyphényle, le N,N-diméthylaminophényle, le 4-acétylaminophényle, le 4-biphényle, le thiényle et le 5-bromothiényle ; en variante, R₄ est choisi parmi le phényle, le 1-naphtyle, le 2-naphtyle, le 4-chlorophényle, le 3-fluorophényle, le 4-fluorophényle, le 4-nitrophényle, le 2-méthylphényle, le 4-propylphényle, le 4-tert-butylphényle, le 2,5-diméthylphényle, le 2,4,6-triisopropylphényle, le 2-dodécylphényle, le 3-dodécylphényle, le 4-dodécylphényle, le 4-méthoxyphényle, le 4-acétylaminophényle, le 4-méthylphényle et le mésityle ; encore en variante, R₄ est choisi parmi le phényle, le 4-chlorophényle, le 4-méthylphényle et le mésityle ;
en variante, dans laquelle R₄ est alkyle en C₁₋₁₂, -ORₐ, -NRₐR_{b}, cycloalkyle en C₃₋₇, ou hétérocyclyle de 3 à 8 chaînons, qui est non substitué ou substitué indépendamment par m' groupe(s) R" ; en variante, dans laquelle R₄ est choisi parmi N,N-diméthylamino, méthyle, éthyle, butyle, dodécyle, méthoxy, éthoxy et cyclopropyle.

3. Méthode selon la revendication 1 ou 2, dans laquelle le cycle A est un phényle, napthyle ou thiényle substitué ou non substitué ; en variante, un phényle substitué ou non substitué.

4. Méthode selon l'une quelconque des revendications 1 à 3, dans laquelle R est H, halogène, alkyle en C₁₋₆, ou halogénoalkyle en C₁₋₆ ; en variante, R est H ou halogène.

5. Méthode selon l'une quelconque des revendications 1 à 4, dans laquelle 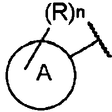 est choisi parmi le phényle, le naphtalèn-1-yle, le naphtalèn-2-yle, le 2-chlorophényle, le 3-chlorophényle, le 4-chlorophényle, le 2-bromophényle, le 3-bromophényle, le 4-bromophényle, le 2,3-dichlorophényle, le 2,4-dichlorophényle, le 2,5-dichlorophényle, le 3,4-dichlorophényle, le 2,3,5-trichlorophényle, le 4-isopropylphényle et le 4-tert-butylphényle ; en variante, 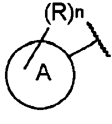 est le 2-chlorophényle.

6. Méthode selon l'une quelconque des revendications 1 à 5, dans laquelle R₁ est choisi parmi les alkyles en C₁₋₆, tels que le méthyle.

7. Méthode selon l'une quelconque des revendications 1 à 6, dans laquelle R₂ est H.

8. Méthode selon l'une quelconque des revendications 1 à 7, dans laquelle R₃ est H, -CH₃, -CH₂CH₃, -CH(CH₃)₂, -C(CH₃)₃, -CH₂CH₂CH₂CH₃, phényle ou - CH₂-phényle ; en variante, R₃ est -CH₃, -CH₂CH₃, -C(CH₃)₃ ou -CH₂-phényle.

9. Méthode selon l'une quelconque des revendications 1 à 8, dans laquelle la base est choisie parmi les bases inorganiques et les bases organiques ; en variante, la base inorganique est choisie parmi les carbonates, les bicarbonates, les phosphates, les hydrogénophosphates, les dihydrogénophosphates, les hydroxydes et les hydrures de métaux alcalins et de métaux alcalino-terreux, par exemple, LiOH, NaOH, KOH, Li₂CO₃, Na₂CO₃, K₂CO₃, Cs₂CO₃, Na₃PO₄, K₃PO₄, K₂HPO₄, KH₂PO₄, et NaH ; en variante, la base organique est choisie parmi les alcoxydes de métaux alcalins et de métaux alcalino-terreux et les amines organiques, tels que NaOMe, KOMe, NaOEt, KOEt, NaOtBu, KOtBu, triéthylamine, DBU, DIPEA, DABCO et DMAP.

10. Méthode selon l'une quelconque des revendications 1 à 9, dans laquelle la réaction est réalisée en la présence d'un solvant choisi parmi l'eau, les alcanes, les éthers, les esters, les alcools, les hydrocarbures halogénés, les cétones, les amides, les sulfones, les sulfoxydes, les nitriles et leurs mélanges, tels que l'eau, le n-heptane, le toluène, le THF, le MTBE, le méthyltétrahydrofurane, l'acétate d'éthyle, l'acétate d'isopropyle, l'acétate de butyle, le méthanol, l'éthanol, l'isopropanol, le tert-butanol, l'alcool tert-amylique, le dichlorométhane, le 1,2-dichloroéthane, le chlorobenzène, l'acétone, la 2-butanone, le DMF, la DMA, la NMP, le DMSO et l'acétonitrile.

11. Méthode selon l'une quelconque des revendications 1 à 10, dans laquelle la température de réaction va de la température ambiante à la température de reflux du solvant, en variante de 40 à 90°C ; encore en variante de 50 à 80°C.

12. Méthode selon l'une quelconque des revendications 1 à 11, dans laquelle le rapport molaire du composé de formule générale (II) au composé de formule générale (III) est de 1 : (0,7-3), en variante 1 : (0,7-1,5), encore en variante 1 : (1-1,2), toujours en variante 1 : 1, 1 : 1,05, 1 : 1,1, 1 : 1,15 ou 1 : 1,2.

13. Méthode selon l'une quelconque des revendications 1 à 12, dans laquelle le rapport molaire du composé de formule générale (II) à la base est de 1 : (1-5), en variante 1 : (2-4), encore en variante 1 : 1,5, 1 : 1,6, 1 : 1,7, 1 : 1,8, 1 : 1,9, 1 : 2, 1 : 2,1, 1 : 2,2, 1 : 2,3, 1 : 2,4, 1 : 2,5, 1 : 2,6, 1 : 2,7, 1 : 2,8, 1 : 2,9 ou 1 : 3.
